**Europäisches Patentamt**

**European Patent Office**

**Office européen des brevets**

(19)

(11) Veröffentlichungsnummer: **0 408 743 A1**

# EUROPÄISCHE PATENTANMELDUNG
## veröffentlicht nach Art. 158 Abs. 3 EPÜ

(12)

(21) Anmeldenummer: 89901923.6

(22) Anmeldetag: 04.11.88

(86) Internationale Anmeldenummer:
PCT/SU88/00221

(87) Internationale Veröffentlichungsnummer:
WO 89/09545 (19.10.89 89/25)

(51) Int. Cl.5: **A23B 7/00**

(30) Priorität: 15.04.88 SU 4413725

(43) Veröffentlichungstag der Anmeldung:
23.01.91 Patentblatt 91/04

(84) Benannte Vertragsstaaten:
**AT BE CH DE FR IT LI NL**

(71) Anmelder: **ORENBURGSKY GOSUDARSTVENNY MEDITSINSKY INSTITUT ul. Sovetskaya 6 Orenburg, 460834(SU)**

(72) Erfinder: **NIKITENKO, Vyacheslav Ivanovich ul. Volodarskogo, 27-53 Orenburg, 460000(SU)**
Erfinder: **NIKITENKO, Ljubov Ivanovna ul. Volodarskogo, 27-53 Orenburg, 460000(SU)**

(74) Vertreter: **von Füner, Alexander, Dr. et al Patentanwälte v. Füner, Ebbinghaus, Finck Mariahilfplatz 2 & 3; 3 D-8000 München 90(DE)**

(54) MITTEL ZUM KONSERVIEREN VON GEMÜSE, OBST UND VERWERTUNGSPRODUKTEN DERSELBEN.

(57) Das Mittel zum Konservieren von Gemüse, Obst und Verwertungsprodukten derselben besteht aus Zellen wenigstens eines Mikroorganismus von Bakterien der Gattung Bacillus und enthält 100 bis $2.10^9$ lebende Zellen je 1 mg Mittel.

EP 0 408 743 A1

# MITTEL ZUM KONSERVIEREN VON GEMÜSE, OBST UND VERWERTUNGSPRODUKTEN DERSELBEN

## Gebiet der Technik

Die vorliegende Erfindung bezieht sich auf die Lebensmittelindustrie und insbesondere auf ein Mittel zum Konservieren von Gemüse, Obst und Verwertungsprodukten derselben.

## Zugrundeliegender Stand der Technik

Zur Zeit wird die Haltbarkeit von Lebensmitteln nach den verschiedenen weit bekannten Verfahren, und zwar durch Kälte und Hitze, Trocknen, Zusatz von Zucker und Salz, chemischen Konservierungsmitteln u.a.m. erreicht.

Die angegebenen Konservierungsmittel sichern die Produktgüte wegen schneller Zerstörung von biologisch wirksamen Stoffen im Produkt nicht.

Das Sterilisieren bei Temperaturen von 108 bis 120°C bewirkt die bedeutenden Strukturveränderungen im Produkt, den Zerfall von Vitaminen, Fermenten, die Verschlechterung der organoleptischen Eigenschaften.

Das Pasteurisierun bei einer zwischen 65 und 100°C liegenden Temperatur sichert die Erhaltung des Nährwertes von Lebensamitteln, ermöglicht jedoch nicht, die fremde Mikroflora völlig zu vernichten, was zum Vederben der Lebensmittel führt. Das Konservieren durch Kühlen erfordert einen hohen Aufwand an Energie und Ausrüstung, bereitet Transportschwierigkeiten.

Die Vewwendung von chemiachen Konservierungsmitteln verschlechtert die Geschmackseigenschaften von Lebensmitteln, führt zum Auftreten der Dysbakterie und Lebensmittelallergie, besonders beim Einsatz von Antibiotika und Antiseptika mit breitem Wirkungsspektrum. Eine ungünstige Einwirkung auf den Organismus übt auch der hohe Gehalt an Salz und Zucker von Konserven aus. Die hohen Salzkonzentrationen verschlechtern die Güte von Lebensmitteln und beschränken ihren Gebrauch.

Bekannt sind mikrobielle Konservierungsmittel, welche darauf basieren, dass Pilze oder Bakterien die Milchsäure als Konservierungsmittel bilden (Salzen, Einsäuern). Dabei wird der pH-Wert herabgesetzt und die Entwicklung vieler Mikroorganismen gestoppt.

Bekannt sind ferner Konservierungsmittel auf der Basis der Reinkulturen von Milchsäurebakterien, die zum Einsäuern von Tomaten und Gurken verwendet werden (G.I.Pushkina. Auswahl von Milchsäurebakterienstämmen zum Einsäuern von Tomaten und Gurken. G.I.Pushkina. Podbor shtammov molochno-kislykh baktery dlya solenia tomatov i ogurtsov. Zeitschrift "Konservnaya i ovoschesushilnaya promyshlennost", 1983 (Moskau), 9, S. 26 bis 27).

Die angegebenen Konservierungsmittel weisen kurze Lagerzeiten und ein beschränktes Anwendungsgebiet auf. Lebensmittelkonserven können nur in einem Temperaturbereich von 0 bis plus 8°C aufbewahrt werden. Bei höheren Temperaturen lösen die Bakterien die Zersetzung von Gemüse und Obst aus. Diese Konservierungsmittel sind bei der Herstellung von Kompotten und Säften nicht zu verwenden.

## Offenbarung der Erfindung

Der Erfindung liegt die Aufgabe zugrunde, durch Auswahl einer Bakterienkultur ein Mittel zum Konservieren von Gemüse, Obst und Verwertungsprodukten derselben zu schaffen, das möglich macht, die hohe Produktgüte zu erreichen, die Lagerzeit des Produkts zu verlängern und sein Anwendungsgebiet zu erweitern.

Die Aufgabe ist dadurch gelöst worden, dass das erfindungsgemässe Mittel zum Koservieren von Gemüse, Obst und Verwertungsprodukten derselben auf der Bakterienbasis erfindungsgemäss aus Zellen wenigstens eines Mikroorganismus von Bakterien der Gattung Bacillus (aus Zellen wenigstens einer Bakteriengattung besteht und 100 bis $2 \cdot 10^9$ lebende Zellen je 1 mg Mittel enthält.

Das erfindungsgemässe Mittel enthält erfindungsgemäss als Bakterien vorteilhaft folgende Ausführungsformen von Stämmen:

- Stamm Bacillus subtilis 534, welcher am 28.03.88 in der Allunions-Kulturensammlung des Instituts für Biochemie und Physiologie von Mikroorganismen der Akademie der Wissenschaften der UdSSR deponiert und unter Nummer B-1666 Д registriert ist;

- Stamm Bacillus sp. 538, welcher am 20.06.88 in der Allunions-Kulturensammlung des Allunions-Forschungsinstituts für Genetik und Selektion von industriellen Mikroorganismen deponiert und unter Nummer BKIM -4401 registriert ist;

- Stamm Bacillus pulvifacieni 535, welcher am 14.04.88 in der Allunions-Kulturensammlung von industriellen Mikroorganismen des Allunions-Forschungsinstituts für Genetik und Selektion von industriellen Mikroorganismen deponiert und unter Nummer BKIM B -4348 registriert ist.

Bakterien der Gattung Bacillus wachsen in Lösungen von Kochsalz, Zucker und scheiden antibakterielle Stoffe mit breitem Wirkungsspektrum aus, die das Wachstum von anderen Bakterien und Pilzen hemmen. Die erfindungsgemäßen neuen Stämme zersetzen die Lebensmittel wegen physiologischer Merkmale von Bakterien nicht. Sie weisen das beschränkte Wachstum auf und sammeln sich in Lebensmitteln und Konservierungsmitteln in einer 50 Tausend Zellen je 1 cm$^3$ über-steigenden Menge nicht an. Fertige Konserven können deshalb bei Raumtemperatur innerhalb von 1 bis 2 Jahren auch dann gelagert werden, wenn die Kochsalz- und Zuckerkonzentrationen herabgesetzt werden. Die Bakterien des erfindungsgemässen Mittels werden durch Erhitzen auf eine Temperatur von 120°C nicht vernichtet. Bei der Verwendung des erfindungsgemäßen Mittels wird die anzuwendende Temperatur bei Erwärmen zwecks Sterilisation auf 80 bis 100°C herabgesetzt, während die Erhitzungszeiten auf das 1,5- bis 2fache gegenüber den bekannten Verfahren verkürzt werden. Erhalten bleiben die biologisch wichtigen Stoffe, verbessert werden die Geschmackseigenschaften und das Aussehen von Lebensmittelkonserven. Eingespart wird die Energie, die Herstellung wird billiger.

Die hergestellten Lebensmittel weisen eine verminderte Fähigkeit zur Allergiewirkung auf, weil die erfindungsgemäßen Stämme Immunomodulatoren ausscheiden. Dies gestattet, neue Konserven selbst den Menschen zu empfehlen, die sie wegen Lebensmittelallergie früher nicht vertragen konnten.

Beste Ausführungsform der Erfindung

Das erfindungsgemäße Mittel wird wie folgt hergestellt.

Aus dem Organismus von Menschen, Tier oder aus der Umwelt werden Bakterien der Gattung Bacillus isoliert. Geprüft wird die Fähigkeit zur Produktion von antibakteriellen Stoffen mit breiten Wirkungsspaktrum. Man untersucht die biologischen Eigenschaften von Stämmen. Bei fehlender Virulenz, Pathogenität, akuter und chronischer Toxizität, teratogener, onkogener, Allergie- und die Geschmackseigenschaften von Produkten verschlechtender Wirkungen werden die Stämme zur Herstellung des Mittels verwendet. Der ausgewählte Stamm wird auf I,5- bis 2%igem Fleisch-Pepton-Agar bei einer zwischen 36 und 38°C liegenden Temperatur innerhalb von 3 Tagen gezüchtet. Die Bakterienkultur wird mit 0,9%iger Natriumchloridlösung oder 5%iger Saccharoselösung abgespült. Man füllt die erhaltene Bakterienaufschwemmung (I00 Tausend bis 200 Md. lebende Zellen je I cm$^3$) unter Asepsisbedingungen in Glasflakons ab und lyophilisiert innerhalb von 24 bis 48 Stunden unter Temperaturgefälle von minus 40 bis plus 40°C und Druck von 60 bis 70 Pa. Man erhält das Endprodukt. Das ist ein weisses oder hellbraunes Pulver. Die Verunreinigung mit der fremden Mikroflora fehlt. Die Zahl der lebenden Zellen je k mg schwankt zwischen I00 und 2Md.

Als Bakterien der Gattung Bacillus enthält das erfindungsgemässe Mittel vorteilhaft folgende neue Stämme: Stamm Bacillus subtilis 534, Stamm Bacillus sp. 538, Stamm Bacillus pulvifaciens 535. Der neue Stamm Bacillus subtilis 534 ist beim Menschen isoliert und mit folgenden morphologischen Merkmalen und physiologischen Eigenschaften gekennzeichnet.

Das sind Stäbchen. Zellgrösse der eintägige Agarkultur (2-4)x(0,6-0,8) $\mu$m. Bakterien beweglich, bilden Sporen und keine Kapseln. Nach Gram färben sich positiv. Kolonien auf Fleisch-Pepton-Agar rauh, mit unebenem Rand, mit schwach rosa Farbton, Durchmesser von 2 bis I2 mm. Der Stamm vermehrt sich bei einer Temperatur von I5 bis 50°C, Wachstumsoptimum 36 bis 37°C. Spaltet Glukose, Saccharose und Mannit bis Säure. Fermentiert Laktose nicht. Bildet Schwefelwasserstoff und Indol nicht. Scheidet Azetylmethylkarbinol und Katalase aus. Empfindlich gegen Benzylpenizillin, Ampizillin, Erythromyzin, Monomyzin, Linkomyzin, Tetrazyklin, unempfindlich gegen Polymyxin.

Der Stamm produziert das Antibiotikum mit breitem Wirkungsspektrum, welches das Wachstum von Staphylokokken, Streptokokken, Proteus, Pyozyaneusbakterium, Hefepilzen u.a. hemmt. Er scheidet ausserdem proteolytische Fermente, welche die tierischen Eiweisse spalten, und Immunomodulator aus.

Der neue Stamm Bacillus sp. 538 wurde beim Menschen isoliert. Das sind Stäbchen. Die Zellgrösse der eintägigen Agarkultur (5-I0)x(0,7-I,2) $\mu$m. Wachsen als Ketten aus 3 bis 5 Zellen. Bewegliche, nach Gram färben sich positiv. Sporen liegen subterminal, bilden keine Kapseln. Kolonien auf Fleisch-Pepton-Agar sind leicht gewölbt, gelblich, von 2 bis 4 mm Durchmesser. Wachsen auf Fleisch-Pepton-Bouillon als Oberflächenhaut und Bodenflocken. Wachsen in 7%igem Natriumchlorid, unter aeroben und anaeroben Bedingungen bei einer Temperatur von 25 bis 40°C, Optimum von 36 bis 37°C. Fermentieren Laktose, Glukose, Saccharose, Mannit, Arabinose, Xylose nicht. Scheiden Schwefelwasserstoff, bilden kein Indol.Koagulieren das Plasma nicht, kein positiver dermatonekrotischer Test.

Der Stamm ist gegen Erythromyzin, Oleandomyzin, Methizillin, Karbenzillin , Ristomyzin, Chloramphenikol empfindlich und gegen Polymyxin, Linkomyzin, Monomyzin beständig.

Der Stamm produziert einen antibakteriellen Stoff mit breitem Wirkungsspektrum.

Der Stamm scheidet ins Aussenmedium proteolytische Fermente aus, welche Albumin, Fibrin, Hämoglobin spalten. Er produziert immunomodulierende Stoffe, welche die Allergisierung abschwächen.

Der Stamm ist untoxisch und apathogen.

Der neue Stamm Bacillus pulvifaciens 535 wurde beim Menschen isoliert. Das sind Stäbchen, Zellgrösse der eintägigen Agarkultur (2-4)x(0,6-0,8) $\mu$m. Bakterien sind beweglich, bilden beim Wachsen auf Fleisch-Pepton-Bouillon ein faltiges Oberflächenhäutchen. Bilden Sporen und keine Kapseln. Färben sich nach Gram positiv. Kolonien auf Fleisch-Pepton-Agar sind rauh, mit unebenem Rand, von schwach rosa Farbton, Durchmesser von 2 bis 9 mm, wachsen sowohl unter aeroben als auch anaeroben Bedingungen. Vermehren sich bei einer Temperatur von I5 bis 50°C. Wachstumsoptimum bei 36 bis 37°C. Spalten bis Säure ohne Gasabscheidung Glukose, Saccharose, Maltose, Dulzit, Galaktose, Fermentieren Laktose, Mannit, Xylose, Rhamnose, Lysin, Arginin, Ornithin nicht. Bilden keinen Schwefelwasserstoff und kein Indol. Peptonisieren die Milch nicht. Produzieren keine Lezithinase. Scheiden Azetylmethylkarbinol und Katalase aus, hydrolysieren die Stärke nicht.

Der Stamm ist gegen Erythromyzin, Ampizillin, Methizillin, Benzylpenizillin, Oxazillin, Linkomyzin, Meomyzin, Chloramphenikol, Monomyzin, Ristomyzin, Kanamyzin, Tetrazyklin, Oleandomyzin empfindlich und gegen Polymyxin unempfindlich.

Der Stamm Bacillus pulvifaciens 535 produziert proteolytische Fermente, welche Albumin, Hämoglobin, Fibrin spalten, Antibiotikum mit breitem Wirkungsspektrum und Immunomodulator. Gegen Antibiotikum sind bedingt pathogene Mikroorganismen empfindlich: Staphylokokken, Streptokokken, Kolibakterien, Pyozyaneusbakterien, Proteus, Salmonellen, Dysenteriebakterien, Klebsiellen, Hefepilze. Hemmt das Wachstum der saprophytierenden Mikroflora nicht. Das Antibiotikum zerfällt ausserdem schnell (innerhalb von einigen Stunden) völlig, was die Entwicklung des Zustands der Lebensmittelallergie verhindert. Die proteolytischen Fermente begünstigen die Assimilation von Nahrungsstoffen.

Hauptunterschiede des angegebenen Stammes vom Stamm Bacillus subtilis 534 bestehen darin, dass die Hydrolyse der Stärke und der Abbau von Mannit nicht auftreten.

Der Stamm ist untoxisch und apathogen.

Untersucht wurde der Antagonismus der erfindungsgemässen Stämme gegen Erreger der bakteriellen Infektionen.

Der Antagonismus des Stammes Bacillus subtilis 534 gegen Erreger der bakteriellen Infektion wurde nach der von W.I.Nikitenko ausgearbeiteten Methodik bestimmt. Kulturröhrchen mit darin befindlichen 5 cm$^3$ steriler Kohlbach-Nährbouillon wurden mit je 40 bis 50 Mio Zellen einer Testkultur (Kontrolle) und mit den gleichen Dosen der Testkultur zusammen mit dem angegebenen Stamm beimpft. Kulturröhrchen wurden in einem Thermostat innerhalb von 24 Stunden bei einer Temperatur von 37°C gehalten. Da die Stammbakterien Bacillus subtilis 534 die Nährbouillondichte unbedeutend beeinflussten, so bestimmte man den Grad der Wachstumshemmung der Testkultur mit Hilfe eines lichtelektrischen Kolorimeters nach dem Bouillondichteunterschied in Versuch und Kontrolle. Als positiv galt das Resultat, wenn die Dichte der Mischkultur auf das I,2fache und darüber geringer als die Bouillondichte der Testkultur war.

Der Stamm Bacillus subtilis 534 hemmte im Wachstum 34 von 37 untersuchten Stämmen des Staphylokokkus, II von I2 Stämmen des Streptokokkus, 8 von I2 Stämmen des Olibakteriums, 7 von 8 Stämmen des Dysenteriebakteriums, 7 bon 7 Stämmen der Salmonellagruppe, 6 von 6 Stämmen des Proteus, 7 von 8 Stämmen des Pyozyaneusbakteriums. Als beständig und weniger empfindlich erwiesen sich hauptsächlich die saprophytierenden Mikroorganismenstämme.

Um die Produktion der antibakteriellen Substanzen ins Aussenmedium nachzuweisen, züchtete man die Stammkulturen Bacillus subtilis 534 (3 Laborreihen) innerhalb von 96 Stunden in Erlenmeyer-Kolben auf Schütteltischen (240 U/min) bei einer Temperatur von 28°C auf einem Nährmedium folgender Zusammensetzung in Gew.%: Erbsenmehl I,5, Saccharose 2,I, Stärke 0,85, NaNO$_3$ 0,5, CaCO$_3$ 0,5, NaCl 0,5, Wasser alles übrige. Die antibakterielle Wirksamkeit von O,I cm$^3$ Filtrat der Kulturflüssigkeit wurde durch Diffusion in den 2%igen Fleisch-Pepton-Agar bestimmt. In Betracht gezogen wurde die Grösse der Hemmungszonen beim Wachsen von Testkulturen unter Substrahieren des Lochdurchmessers (8 mm).

Die Wachstumshemmungszonen bei Staphylokokkus betrugen 24 bis 27 mm, bei Kolibakterium I8 bis 22 mm, bei Klebsiella I8 bis 2I mm, bei Hefepilz 22 bis 24 mm.

In in-vitro-Versuchen hemmte der Stamm Bacillus sp. 538 im Wachstum II bon I2 untersuchten Stämmen des Staphylokokkus, 5 bon 6 Stämmen von Streptokokken und 7 von 9 Stämmen von Salmonellen. Wenn nach der Methode der Diffusion in den Agar unter Verwendung der Testkulturen und der Kulturflüssigkeit gearbeitet wurde, wurde das Wachstum von Staphylokokkus in einer Zone von 35,I±0,3 mm, von Kolibakterium in einer Zone von 22,0±0,2 mm, von Klebsiella in einer Zone von 20,9±0,2 mm, von Hefepilz in einer Zone von 28,0±0,2 mm gehemmt. Bei Verdünnung von I:I0 betrugen die Wachstumshemmungszonen bei Staphylokokkus 27,9±0,I mm, bei Kolibakterium 20,0±0,I mm, bei Klebsiella 20,2±0,2 mm, bei Hefepilz 29,2±0,2 mm. Das vom Stamm produzierte Antibiotikum erhielt seine Wirksamkeit in der gleichen Lösung bei Raumtemperatur innerhalb von 3 Tagen.

Der Stamm Bacillus pulvifaciens 535 hemmte im Wachstum 34 von 37 untersuchten Stämmen von Staphylokokkus, 12 von 12 Stämmen von Streptokokkus, 12 von 12 Stämmen von Kolibakterium, 7 von 8 Stämmen von Dysenteriebakterium, 7 von 7 Stämmen von Salmonella, 6 von 6 Stämmen von Proteus, 7 von 8 Stämmen von Pyozyaneusbakterium. Als beständig und weniger empfindlich erwiesen sich hautpsächlich die  saprophytierenden Mikroorganismenstämme. Das vom Stamm produzierte Antibiotikum zerfiel in einer wässrigen Lösung innerhalb von einigen Stunden.

Untersucht wurde die Produktion der Proteasen von den erfindungsgemässen Stämmen ins Aussenmedium.

Die Produktion der proteolytischen Fermente ins Aussenmedium bei 3 Laborreihen des Stammes Bacillus subtilis 534 wurde nach der Ansson-Methode ermittelt.

Die Prüfergebnisse bei allen 3 Reihen haben ergeben, dass der Stamm eine Fähigkeit besitzt, albumin-, hämoglobin-, fibrinspaltende Fermente zu produzieren.

Neben Antibiotika produzieren die Stämme Bacillus pulvifaciens 535 und Bacillus sp. 538 exogen proteolytische hämoglobin- und albuminspaltende Fermente. Das Vorhandensein der Proteasen wurde ebenfalls nach der Ansson-Methode mit lebenden eintägigen Kulturen bestimmt.

Untersucht wurde ebenfalls die Produktion der Immunomodulatoren von den erfindungsgemässen Stämmen. Die 3tägige Kulturflüssigkeit in einer Menge von 0,1 cm$^3$ ist nach der Entfernung von Bakterienstämmen Bacillus subtilis 534 und Bacillus sp. 538 bei der Reaktion der Blasttransformation auf ähnliche Weise wie Phytohämagglutinin wirksam. Es wurden je 6 Versuche durchgeführt.

Die Menge von gebildeten Blasten beträgt in Kontrolle 0,05%, im Falle der Kulturflüssigkeit des Stammes Bacillus subtilis 534 42±2% und der des Stammes Bacillus sp. 538 54±2%.

Bei der Prüfung der akuten Toxizität des erfindungsgemässen Mittels wurden 3 Laborreihen des aus der lebenden Kultur des Stammes Bacillus subtilis 534 hergestellten Präparats verwendet. Jede Reihe wurde 3 weissen Mäussen und 3 weissen Wistar-Ratten in einer Dosis von IO bzw. 20 Md. Zellen je I cm$^3$ 0,9%ige NaCl-Lösung einmal intraperitoneal appliziert. Alle Tiere blieben am Leben. Sie assen gut Futter. Die Tiere wurden am 3., 7., I4. Versuchstag geschlachtet. Bei der histologischen Untersuchung sind keine entzündlichen und dystrophischen Änderungen von Gehirn, Lungen, Nieren, Leber und Herzen nachgewiesen. In der Milz treten vergrösserte Follikel und höhere Mengen von lymphohistiozytären Elementen in der roten Pulpa auf.

Zwecks Ermittlung des Grades der akuten Toxizität wurden 3 Präparatreihen aus dem Stamm Bacillus subtilis 534 je mit der Nahrung von 6 weissen Mäusen und 6 weissen Wistar-Ratten in einer Dosis von IO bzw. 20 Md. Zellen einmal eingenommen. Alle Tiere blieben am Leben. Am nächsten Tag wurden sie geschlachtet. Bei der histologischen Untersuchung von Gehirn, Myokard, Lungen, Nieren, Magen, Dünn- und Dickdarm sind die Änderungen gegenüber Tieren der Kontrollgruppe (IO Mäuse und IO Ratten) nicht nachgewiesen. Die Leben zeigt eine gewisse Vermehrung der Menge von lymphohistiozytären Infiltraten in der Richtung von Portaltrakten. In der Milz treten vergrösserte Follikel und grosse Mengen von lymphohistiozytären Elementen in der roten Pulpa und vielkernigen Riesenzellen auf.

20 weissen Mäusen wurden mit Formalindämpfen vorgetötete Stämme Bacillus pulvifaciens 535 und Bacillus sp. 538 in einer Dosis von 2 Md. Zellen je I cm$^3$ 0,9%ige NaCl-Lösung intraperitoneal injiziert. Noch 30 Mäusen und 30 weissen Wistar-Ratten wurden lebende Kulturen der gleichen Stämme in einer Dosis von IO bzw. 20 Md. Zellen eingespritzt. Alle Tiere blieben am Leben. Sie assen gut Futter. Die Tiere wurden am 3., 7. und I4. Versuchstag geschlachtet. Bei der histologischen Untersuchung sind keine entzündlichen und dystrophischen Änderungen von Gehirn, Lungen, Nieren, Herzen und Leber nachgewiesen. In der Milz treten vergrösserte Follikel und höhere Mengen von lymphohistozytären Elementen in der roten Pulpa auf.

I5 weisse Mäuse und I5 weisse Wistar-Ratten nahmen peroral je IO bzw. 20 Md. Aufschwemmungszellen der lebenden lyophilisierten Stammkultur Bacillus pulvifaciens 535 ein. Die Tiere blieben am Leben. Am nächsten Tag wurden sie geschlachtet. Bei der histologischen Untersuchung
von Gehirn, Myokard, Lungen, Nieren, Magen, Dünn- und
Dickdarm sind keine Änderungen gegenüber Tieren der Kontrollgruppe nachgewiesen. Die Leber zeigt eine gewisse
Vergrösserung der Menge von lymphohistiozytären Infiltraten in der Richtung von Portaltrakten. In der Milz treten
vergrösserte Follikel und höhere Mengen von lymphohistiozytären Elementen in der roten Pulpa und vielkernigen
Riesenzellen auf.

I2 Kaninchen wurden subkutan lebende eintägige Stammkulturen Bacillus pulvifaciens 535 und Bacillus sp. 538
je in einer Dosis von IOO Tausend, I Mio, IO Mio, I Md.
und 5 Md. Zellen je I cm$^3$ 0,9%ige NaCal-Lösung injiziert.
Der Allgemeinzustand von Tieren veränderte sich nicht,
sie alle blieben am Leben. Örtliche Änderungen an der
Injektionsstelle waren nicht nachgewiesen, aber die sporenbildenden Bakterien traten aus der Infektionszone innerhalb von 2 Wochen aus.

Bei der Prüfung der chronischen Toxizität wurden 3
Präparatreihen aus dem Stamm Bacillus subtilis 534 je
innerhalb von 30 Tagen 3 weissen Mäusen und 3 weisen Wistar-Ratten intraperitoneal in einer Dosis von 200 Mio bzw.
I Md. Zellen je I cm$^3$ 0,9%ige NaCl-Lösung injieziert. Jede der drei Präparatreihen wurde ausserdem in den gleichen
Dosen von 6 weissen Mäusen und 6 weissen Wistar-Ratten
eingenommen. Alle Tiere blieben am Leben. Der Gewichtszuwachs von Versuchstieren betrug gegenüber Kontrolltieren (IO Mäuse und IO Ratten) II bis I7% (p< 0,05). Das
Haar bei Mäusen und Ratten der Versuchsgruppen war grellweiss und weich. Die Tiere wurden am 3I. Tag geschlachtet.
Bei der histologischen Untersuchung von Gehirn, Myokard,
Nieren, Lungen, Magen, Dünn- und Dickdarm sind die Änderungen nicht nachgewiesen. In der Leber fanden sich lymphohistiozytäre Infiltrate in der Richtung von Portalkanälen in einer grösseren Menge gegenüber Tieren der Kontrollgruppen. In der Milz von Versuchstieren beobachtet
man die Lymphoidisation der roten Pulpa und vergrösserte
Follikel, aber die vielkernigen Riesenzellen treten nicht
auf.

Bei den Versuchen mit dem Ziel, die chronische Toxizität zu prüfen, wurden bei 6 weissen Mäusen 23 Mäuschen ohne Abnormität am II. bis 2I. Tag geboren. Die letzteren brachten dann 7 Mäuschen ohne Abnormität zur Welt.

Bei der Prüfung der chronischen Toxizität wurden die lebenden eintägigen Stammkulturen Bacillus pulvifaciens 535 und Bacillus sp. 538 täglich innerhalb von 30 Tagen intraperitoneal 40 weissen Mäusen in einer Dosis von 200 bis 500 Mio. Zellen und 30 Wistar-Ratten in einer Dosis von 2 Md. Zellen je I cm$^3$ 0,9%ige NaCl-Lösung eingespritzt. 48 weisse Mäuse und 30 Wistar-Ratten nahmen ausserdem mit Wasser täglich innerhalb von 30 Tagen je 200 Mio und I Md. Zellen der lebenden lyophilisierten Kultur der angegebenen Stamme ein. Alle Tiere blieben am Leben. Es sei bemerkt, dass der Gewichtszuwachs von Versuchstieren gegenüber Kontrolltieren II bis I9% betrug. Das Haar bei Mäusen und Ratten der Versuchsgruppe war grellweiss und weich. Die . Tiere wurden geschlachtet. Bei der histologischen Untersuchung von Gehirn, Myokard, Nieren, Lungen, Magen, Dünn- und Dickdarm sind die Änderungen nicht nachgewiesen. In der Leber fanden sich lymphohistiozytäre Infiltrate in einer höheren Menge gegenüber Kontrolltieren sowohl in der Richtung von Portalkanälen als auch ausserhalb derselben. In der Milz von Tieren der Versuchsgruppe beobachtete man die Lymphoidisation der roten Pulpa und vergrösserte Follikel, aber die vielkernigen Riesenzellen traten nicht auf.

Im Laufe der Durchführung der Versuche mit dem Ziel, die chronische Toxizität zu untersuchen, wurden bei 9 weissen Mäusen 54 Mäuschen ohne Abnormität am II. bis 2I. Tag geboren. Die letzteren brachten dann 9 Mäuse ohne Abnormität zur Welt. Die lebenden Stammkulturen beeinflussen also die Entwicklung der Leibesfrucht selbst bei intraperitonealer Injektion nicht und rufen keine genetischen Störungen hervor.

3 Präparatreihen aus dem Stamm Bacillus subtilis 534 wurden je in einer Dosis von 2 Md. Zellen je I cm$^3$ 0,9%ige NaCl-Lösung sechsmal jeden zweiten Tag 4 Meerschweinchen eingespritzt. Am 3I. Tag wurden allen Meerschweinchen subkutane Injektionen des Präparats in einer Dosis von 2 Md. Zellen je I cm$^3$ 0,9%ige NaCl-Lösung gemacht. Örtliche und allgemeine Manifestationen der allergischen Reaktion traten nicht auf.

Die lebenden Stammkulturen Bacillus pulvifaciens 535 und Bacillus sp. 538 wurden in einer Dosis von 2 Md. Zellen je I cm$^3$ 0,9%ige NaCl-Lösung sechsmal jeden zweiten Tag 24 Meerschweinchen (I Stamm für I2 Tieren) subkutan appliziert. Am 3I. Tag wurden allen Meerschweinchen intrakutane Injektionen von 2 Md. Zellen der Mikroorganismen der gleichen Stämme je I cm$^3$ 0,9%ige NaCl-Lösung gemacht. Örtliche und allgemeine Manifestationen der allergischen Reaktion traten nicht auf.

Zwecks Bestimmung der Stabilität des erfindungsgemässen Mittels wurden 9 Kapseln Präparat, hergestellt aus Stämmen Bacillus subtilis 534, Bacillus sp. 538, Bacillus pulvifaciens 535, in dicht abgeschlossenen Flakons bei Raumtemperatur innerhalb von 7 Jahren gelagert. Noch 9 Kapseln wurden innerhalb von 3 Monaten bei einer Temperatur von minus 20 bis 22°C, 9 Kapseln innerhalb von 2 Monaten bei einer Temperatur von II0°C aufbewahrt. Die Menge von lebenden Bakterien in Kapseln ermittelte man durch Inokulation bei Serienverdünnungen.

Vor Versuchsbeginn waren in Kapseln 5,4±0,5 Md. Bakterienzellen, bei Lagerung innerhalb von 7 Jahren 5,2±0,5 Md. Bakterienzellen, bei Lagerung bei einer Temperatur von minus 20 bis 22°C 5,4±0,6 Md. Bakterienzellen, bei Lagerung bei einer Temperatur von 110°C 5,3±0,6 Md. Bakterienzellen, enthalten. Die gewonnenen Angaben zeugen also davon, dass das erfindungsgemässe Mittel innerhalb von etwa 7 Jahren in einem weiten Temperaturbereich aufbewahrt werden kann.

Das erfindungsgemässe Konservierungsmittel wird als Pulver hergestellt und lässt sich dosieren. Bei fehlender Feuchte kann es innerhalb von 5 bis 7 Jahren in einem weiten Temperaturbereich aufbewahrt werden. Die Aufwandmenge des erfindungsgemässen Konservierungsmittels ist gering und beträgt 0,0001 bis 1 g je 1 dm$^3$ Produkt.

Beim Konservieren legt man das gewaschene Gemüse und Obst in Gläser ein und übergiesst mit Wasser, welches Geschmack-Zutaten (Salz, Zucker, Gewürze und anderes mahr) enthält. Säfte und Kompotte werden nach der bekannten Technologie zubereitet. Den Gläsern mit dem Füllgut setzt man das erfindungsgemässe Konservierungsmittel ausgehend

von IOO bis I Md.Zellen je I dm$^3$ zu. Die Gläser werden mit Metalldeckeln abgeschlossen und bei einer Temperatur von 80 bis IOO°C innerhalb von IO bis 30 Minuten pasteurisiert. Dann werden die Konserven bei Raumtemperatur aufbewahrt.

Durch Zusatz von weniger als IOO Bakterien je I dm$^3$ kann die Konservierungsstoffwirkung nicht erreicht werden. Eine I Md. Zellen je I dm$^3$ übersteigende Dosis ergibt keinen höheren positiven Effekt.

Durchgeführt wurden die Gebrauchstests an mit Hilfe des erfindungsgemässen Mittels konservierten Produkten.

Die hergestellten Produkte wurden zur Ernährung von 9 Kranken und 8 gesunden Menschen verwendet. 3 Patienten litten an Dysbakterie, 4 an Diathese, Kinderexzem, Lebensmittelallergie, 2 Patienten hatten eitrige Wunden von Extremitäten.

Die Probanden eregistrierten hohe Geschmackseigenschaften von Konserven. Im Verlauf eines Beobachtungsjahres waren keine pathologischen Reaktionen zu verzeichnen. Ohne zusätzliche Behandlung trat hingegen die Genesung von 8 Kranken auf. Beim Kranken mit der eitrigen Wunde der Extremität kam es zur Zustandsbesserung, was als Reinigung von nekrotisierten Geweben und Randepithelisation zutage getreten war.

Es gab keine allergischen Reaktionen selbst bei jenen Kranken, die den Hautausschlag nach der beschränkten Einnahme der gleichen nach der bekannten Technologie hergestellten Konserven registrierten.

Zum besseren Verstehen der vorliegenden Erfindung werden folgende Beispiele für die Herstellung und Anwendung des erfindungsgemässen Konservierungsmittels angeführt.

Beispiel I

Der Stamm Bacillus subtilis 534 wird in 500 cm$^3$ grosse Glasgefässe mit darin befindlichem 2%igem Fleisch-Pepton-Agar inokuliert, welche dann in einem Thermostat bei einer Temperatur von 37°C für 72 Stunden eingebracht werden. Dann spült man die Kultur mit 0,9%iger NaCl-Lösung ab und führt die Lyophilisation des gewonnenen Produkts innerhalb von 24 Stunden bei einem Temperaturgefälle von minus 40 bis plus 40°C und einem Druck von 60 bis 70 Pa durch. Man erhält 2,4 g hellbraunes Pulver mit etwa I Md. lebenden Zellen je I mg. Das erhaltene Mittel wurde zum Konservieren von Gurken verwendet. Die Gurken werden gewaschen und mit fliessendem Wasser innerhalb von 30 Minuten geweicht. Man bereitet eine Salzlösung zu, welche in Masse% enthält: NaCl 3,0, Essigsäure 0,5, Dill I,0, Petersiliengrün 0,025, schwarzen Pfeffer 0,04, Paprika 0,07. Die Gurken werden in 20 Gläser von I dm$^3$ Fassungsvermögen eingelegt und mit der Salzlösung übergossen. In I5 Gläser bringt man je I mg Konservierungsmittel ein, 5 Gläser ohne hergestellte Konservierungsmittel dienen als Kontrolle. Alle Gläser werden mit Metalldeckeln abgeschlossen und eingewalzt. Die Pasteurisation erfolgt bei einer Temperatur von 80°C innerhalb von IO Minuten. Bei der nach 2 Monaten durchgeführten Kontrolle sind die Gurken aus den Gläsern, in denen das erfindungsgemässe Konservierungsmittel verwendet wurde, olivfarben, beim Schneiden dicht, knirschen und schmecken nach leicht gesalzenen. Der Gehalt an Vitamin C beträgt 4,8 mg je IOO g Gurken. Alle Gläser wurden bei Raumtemperatur (I8 bis 20°C) gelagert. Die Gurken in 5 Kontrollgläsern (ohne Konservierungsmittel) wurden nach 4 bis 6 Tagen wegen Entwicklung der Mikroflora unbrauchbar.

Bei der Lagerung der unter Verwendung des erfindungsgemässen Mittels hergestellten Konserven innerhalb von I Jahr war die Verderbnis nicht zu verzeichnen. Die organoleptischen Eigenschaften von Gurken blieben unverändert. die Salzlösung ist klar. Die bakteriologische Untersuchung hat ergeben, dass etwa 50 Tausend lebende Bakterienzellen des Stammes Bacillus subtilis 534 je I cm$^3$ Salzlösung enthalten sind.

Beispiel 2

Man erhält das Konservierungsmittel analog zu Beispiel I. Es wird zum Konzervieren von Gewächshaus-Gurken verwendet. Das Konservieren erfolgt analog zu Beispiel I. Man erhält 20 Gläser Konserven, die innerhalb von II Monaten bei einer zwischen I5 und 25°C liegenden Temperatur aufbewahrt werden. In geöffneten Gläsern hatten die Gurken einen schwach gesalzenen Geschmack, waren dicht und knirschten. In Kontrollgläsern (ohne Verwendung des erfindungsgemässen Mittels-) wurden die Konserven am 2. bis II. Tag
werden Erweichung der Gurken unbrauchbar.

Beispiel 3

Analog zu dem in Beispiel I beschriebenen erhält man
auf der Basis des Stammes Bacillus pulvifaciens 535 2,5 g
Konservierungsmittel, das etwa 500 Tausend lebende Zellen
je I mg enthält. Man benutzt dieses Mittelzzum Konservieren von Tomaten. Reife Tomafe werden gewaschen und in 25
Gläser von 3 dm$^3$ Fassungsvermögen eingelegt. Man bereitet
die Salzlösung zu, die in Masse% enthält: Natriumchlorid
3,0, Dill 0,5, Petersilie 0,3, Meerettich 0,2, Blätter
von Johannisbeere und Kirsche 0,02, Lorbeerblatt 0,00I
enthält. Die Salzlösung wird in Gläser mit Tomaten übergossen. In 20 Gläser bringt man je I mg hergestelltes
Mittel ein, 5 Gläser dienen als Kontrolle. Die Gläser werden abgedeckelt und bei einer Temperatur von 95°C innerhalb von IO Minuten pasteurisiert. Nach 4 bis 49 Tagen
wurden 4 Kontrollgläser von 5 unbrauchbar. Nach 2 Monaten
wurde das fertige Produkt degustiert. Das Produkt weist
ein gutes Aussehen und gute Geschmackseigenschaften auf.
Der Gehalt an Vitamin C beträgt I8,4 mg je I00 g Tomate.
In I cm$^3$ Salzlösung sind etwa I,5 Tausend lebenbe Zellen
des angegebenen Stammes enthalten. Die Konserven wurden
innerhalb von I Jahr bei Raumtemperatur gelagert und erhielten ihre Geschmackseigenschaften und das Aussehen.

Beispiel 4

Analog zu dem in Beispiel I beschriebenen erhält man
ein Konservierungsmittel auf der Basis von Stamm Bacillus
pulvifaciens 535. Es wird zum Konservieren des Birnensaftes verwendet. Der Birnensaft wird durch Zerkleinern und
Zentrifugieren hergestellt, in 7 Gläser von 0,5 dm$^3$ Fassungsvermögen abgefüllt. In 4 Gläser bringt man je I mg
hergestelltes Mittel ein, welches etwa 500 Tausend lebende
Zellen je I mg enthält. 3 Gläser dienen als Kontrolle
(ohne Zusatz des Mittels). Die Gläser werden abgedeckelt
und bei einer Temperatur von 80°C innerhalb von 30 Minuten
pasteurisiert. Der gewonnene Saft wurde innerhalb von I4
Monaten bei Raumtemperatur aufbewahrt und erhielt seine

- 16 -

Geschmackseigenschaften. Alle Konserven in Kontrollgläsern wurden unbrauchbar.

Beispiel 5

Analog zu dem in Beispiel I beschriebenen erhält man I,5 g Mittel auf der Basis des Stammes Bacillus sp. 538, das 200 Mio. lebende Zellen je I mg enthält. Das Mittel wird zum Konservieren von Äpfeln verwendet. Gewaschene Äpfel werden in 8 Gläser von I dm$^3$ Fassungsvermögen eingelegt und mit 5%iger Saccharoselösung übergossen. In 4 Gläser mit dem Füllgut bringt man je 5 mg hergestelltes Mittel ein, 4 Gläser dienen als Kontrolle (ohne Zusatz des Mittels). Die Gläser werden abgedeckt und bei einer Temperatur von I00°C innerhalb von I0 Minuten pasteurisiert. Alle unter Verwendung des erfindungsgemässen Mittels hergestellten Konserven erhielten ihre organoleptischen Eigenschaften bei der Lagerung innerhalb von I Jahr. In Kontrollgläsern wurden die Konserven wegen Entwicklung der Mikroflora unbrauchbar.

Beispiel 6

Analog zu dem in Beispiel I beschriebenen erhält man 2,2 g Mittel auf der Basis des Stammes Bacillus sp. 538, das 200 Mio. lebende Zellen je I mg Mittel enthält. Man verwendet dieses Mittel zum Konservieren von Kompotten aus Kirschen. In 5 Gläser mit Kompott aus Kirschen, dessen Fassungsvermögen 0,5 dm$^3$ beträgt, bringt man je I mg genanntes Mittel ein. Die Gläser werden abgedeckt und bei einer Temperatur von I00°C innerhalb von I0 Minuten pasteurisiert. Bei der Lagerung der hergestellten Konserven innerhalb von I Jahr erhielt das Produkt seine organoleptischen Eigenschaften.

Beispiel 7

Analog zu dem in Beispiel I beschriebenen erhält man das Mittel, welches je I mg 50 Mio. lebende Zellen des Stammes Bacillus subtilis 534, 70 Mio. lebende Zellen des Stammes Bacillus pulvifaciens 535, I0 Mio. lebende Zellen des Stammes Bacillus sp. 538 enthält. Das hergestellte Mittel wird zum Konservieren von Gurken nach der in Beispiel I beschriebenen Methodik verwendet.

In I0 Gläser mit Gurken, dessen Fassungsvermögen I dm$^3$ beträgt, bringt man je I mg hergestelltes Mittel ein, 5 Gläser dienen als Kontrolle (ohne Zusatz des Mittels). Die unter Verwendung des erfindungsgemässen Mittels hergestellten Konserven erhielten ihre Geschmackseigenschaften bei der Lagerung derselben innerhalb von I Jahr. In allen 5 Kontrollgläsern wurde die Salzlösung trübe, 3 Kontrollgläser wurden geöffnet.

Industrielle Anwendbarkeit

Das erfindungsgemässe Mittel wird in der Lebensmittelindustrie zum Konservieren von verschiedenem Gemüse, Obst, Säften, Kompotten und sonstigen Verwertungserzeugnissen derselben verwendet. Das erfindungsgemässe Mittel kann bei der Herstellung von Produkten der Diätkost zur Behandlung von Dysbakterie, Diathese, Kinderekzem, eitrigen Entzündungsprozessen zur Verwendung kommen.

PATENTANSPRÜCHE:

1. Mittel zum Konservieren von Gemüse, Obst und Verwertungsprodukten derselben auf der Bakterienbasis, dadurch g e k e n n z e i c h n e t , dass es aus Zellen wenigstens eines Mikroorganismus von Bakterien der Gattung Bacillus besteht und 100 bis $2 \cdot 10^9$ lebende Zellen je 1 mg Mittel enthält.

2. Mittel nach Anspruch 1, dadurch g e k e n n - z e i c h n e t , dass es als Bakterien den Stamm Bacillus subtilis 534 enthält, welcher am 28.03.88 in der Allunions- -Kulturensammlung des Instituts für Biochemie und Physiologie von Mikroorganismen der Akademie der Wissenschaften der UdSSR deponiert und unter Nummer B-1666Д registriert ist.

3. Mittel nach Anspruch I, dadurch. g e k e n n - z e i c h n e t, dass es als Bakterien den Stamm Bacillus sp. 538 enthält, welcher am 20.06.88 in der Allunions-Kulturensammlung von industriellen Mikroorganismen des Allunions-Forschungsinstituts für Genetik und Selektion von industriellen Mikroorganismen deponiert und unter Nummer ВКПМ -440I registriert ist.

4. Mittel nach Anspruch I, dadurch g e k e n n - z e i c h n e t, dass es als Bakterien den Stamm Bacillus pulvifaciens 535 enthält, welcher am I4.04.88 in der Allunions-Kulturensammlung von industriellen Mikroorganismen des Allunions-Forschungsinstituts für Genetik und Selektion von industriellen Mikroorganismen deponiert und unter Nummer ВКПМ -4348 registriert ist.

# INTERNATIONAL SEARCH REPORT

## I. CLASSIFICATION OF SUBJECT MATTER (if several classification symbols apply, indicate all) *

According to International Patent Classification (IPC) or to both National Classification and IPC

IPC⁴ A23B 7/00

## II. FIELDS SEARCHED

### Minimum Documentation Searched 7

| Classification System | Classification Symbols |
|---|---|
| IPC⁴ | A23B 7/00, 7/10, 7/12, 7/14 |

Documentation Searched other than Minimum Documentation
to the Extent that such Documents are Included in the Fields Searched *

## III. DOCUMENTS CONSIDERED TO BE RELEVANT *

| Category * | Citation of Document, 11 with indication, where appropriate, of the relevant passages 12 | Relevant to Claim No. 13 |
|---|---|---|
| A | DE, B2, 2001874 (EDEN-WAREN GmbH) 14 November 1974 (14.11.74) see the claims | 1-4 |
| A | SU, A1, 376078 (Moskovsky institut narodnogo khozyaistva imeni G.V. Plekhanova) 31 May 1973 (31.05.73) see column 1, lines 23-30 | 1-4 |
| A | SU, A1, 644832 (Moldavsky nauchno-issledovatelsky institut pischevoi promyshlennosti et al.) 30 January 1979 (30.01.79) see the claims | 1-4 |

---

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 14 March 1989 (14.03.89) | 25 April 1989 (24.04.89) |

| International Searching Authority | Signature of Authorized Officer |
|---|---|
| ISA/SU | |

Form PCT/ISA/210 (second sheet) (January 1985)